# FASCICULE DE BREVET EUROPEEN

(11) **EP 0 747 379 B1**
(45) Date de publication et mention de la délivrance du brevet: **11.08.1999**
(21) Numéro de dépôt: 96401208.2
(22) Date de dépôt: 06.06.1996
(51) Int. Cl.: C07D 403/06, A61K 31/40, C07D 413/06

(54) **Dérivés de l'indole, de l'indazole et du benzisoxazole pour le traitement de la schizophrénie**
Indol-, Indazol- und Benzisoxazolderivate zur Behandlung von Schizophrenia
Derivatives of indole, indazole and benzisoxazole for the treatment of schizophrenia

(30) Priorité: 07.06.1995 FR 9506663
(43) Date de publication de la demande: 11.12.1996
(73) Titulaire: ADIR ET COMPAGNIE, 92415 Courbevoie Cédex (FR)
(72) Inventeur: Lavielle, Gilbert, 78170 La Celle Saint Cloud (FR); Muller, Olivier, 95300 Ennery (FR); Millan, Mark, 75017 Paris (FR); Audinot, Valérie, 78300 Poissy (FR)

(56) Documents cités:
- DE-A- 2 708 913
- JOURNAL OF MEDICINAL CHEMISTRY vol. 37, no. 15, 1994, WASHINGTON US, XP002012423 N. J. HRIB ET AL. 'Benzisoxazole- and benzisothiazole-3-carboxamides as potential atypical antipsychotic agents'
- JOURNAL OF MEDICINAL CHEMISTRY, vol. 36, no. 21, 1993, WASHINGTON US, pages 3073-3076, XP002012424 R.E.MESHAW ET AL.: "Examination of the D2/5-HT2 affinity ratios ..."
- CHEMICAL ABSTRACTS, vol. 106, no. 25, 22 Juin 1987 Columbus, Ohio, US; abstract no. 213767z, SUGIMOTO,HACHIRO ET AL.: "Piperidine derivatives." XP002012425 & JP-A-61 227 565 (EISAI CO.,LTD.) 09 Octobre 1986

## Description

La présente invention concerne de nouveaux dérivés de l'indole, de l'indazole et du benzisoxazole, leur procédé de préparation et les compositions pharmaceutiques qui les contiennent.

De nombreux dérivés de l'indole ont été décrits dans la littérature. Certains d'entre eux ont été développés en tant qu'agonistes des récepteurs 5-HT₁₋ₗᵢₖₑ pour le traitement et la prévention des douleurs occasionnées par un flux vasculaire anormal comme la migraine et les maladies associées. C'est le cas plus particulièrement des composés décrits dans les brevets EP 382570, DE 3131728, EP 438230 et EP 486666. Le brevet DE 2708913 décrit des dérivés de l'indole ayant des propriétés sédatives, analgésiques et hypotensives. Le brevet EP 135781 décrit, quant à lui, des dérivés de l'indazole en tant qu'analgésiques centraux ayant des propriétés neuroleptiques.
Enfin, le brevet EP 518805 décrit des dérivés de l'indole, de l'indazole et du benzisoxazole qui sont de puissants ligands aux récepteurs sigma.

Les composés de la présente invention, outre le fait qu'ils soient nouveaux, présentent des propriétés pharmacologiques particulièrement intéressantes. En effet, un des défis de la psychopharmacologie aujourd'hui est de trouver de nouveaux médicaments permettant un meilleur contrôle de la schizophrénie dont le traitement est actuellement peu satisfaisant. Les neuroleptiques classiques, tel que l'halopéridol, soignent assez bien les symptômes productifs (tels que la fabulation et les hallucinations) mais leur efficacité vis-à-vis des symptômes déficitaires (tels que le retrait social) est très faible. De plus, ils provoquent un syndrome extrapyramidal de type Parkinsonien comme l'ont indiqué A.Y. DEUTCH et coll. (Schizophrenia Research, 4, 121-151, 1991) et H.Y. MELTZER et coll. (Pharmacol. Rev., 43, 587-604, 1991).

A la différence de l'halopéridol, la clozapine est plus efficace en traitant les symptômes déficitaires et elle est même efficace chez les patients qui résistent à l'halopéridol. De surcroit, elle ne provoque quasiment pas de syndrome extrapyramidal comme l'ont indiqué COWARD et coll. (Psychopharmacology, 99, s6-s12, 1989). Cette différence semble être due au profil réceptoriel de la clozapine qui est différent de celui de l'halopéridol : par exemple, sa faible activité relative sur les récepteurs D₂ et sa relativement forte affinité sur les récepteurs sérotonergiques, plus particulièrement 5-HT_{2A} et 5-HT_{2C}. Ces résultats ont été indiqués par H. CANTON et coll. (Eur. J. Pharmacol., 191, 93-96, 1990), ainsi que par A.Y. DEUTCH et H.Y. MELTZER cités plus haut.

De plus, en ce qui concerne son absence d'effet cataleptogène à la différence de l'halopéridol, la clozapine montre une certaine affinité pour les récepteurs 5-HT_{1A}, dont l'activation est d'ailleurs associée aux effets anxiolytiques, antidépresseurs et, éventuellement même, antipsychotiques comme l'ont indiqué S. AHLENIUS (Pharmacol. & Toxicol., 64, 3-5, 1989), J. E. BARRETT et coll. (in "5-HT_{1A} agonists, 5-HT₃ antagonists and benzodiazepines : their comparative behavioural pharmacology", Ed. R.J. ROGERS and S.J. COOPER, WILEY & Sons Ltd, Chichester, p. 59-105, 1991), M.J. MILLAN et coll. (Drug News Perspectives, 5, 397-466, 1992), J.M.A. SITSEN (DRUG NEWS & Perspectives, 4, 414-418, 1991).

Néanmoins, en raison de sa toxicité, la clopazine ne peut pas être envisagée dans le traitement général des états schizophréniques. Il était donc particulièrement intéressant de trouver des produits partageant le mécanisme de cette dernière mais dépourvus de ses effets toxiques qui sont directement reliés à sa structure chimique particulière.

Les composés décrits dans ce brevet correspondent au profil recherché. En effet, ils présentent (in vitro et in vivo) un profil biochimique et fonctionnel très similaire à celui de la clozapine et avec de surcroit des affinités pour les récepteurs 5-HT_{1A}, 5-HT_{2A} et/ou 5-HT_{2C} plus importantes. Ils possèdent donc un profil original qui semble être bien adapté à un meilleur traitement de la schizophrénie en comparaison de ce qu'il est actuellement possible d'obtenir avec les produits disponibles.

D'autre part, ce profil pharmacologique tout à fait remarquable n'a pas été obtenu avec les composés les plus proches de l'art antérieur décrits par N.J. HRIB et coll. (J. Med. Chem., 37, 2308-2314, 1994) qui possèdent dans leur structure un cycle pipéridine à la place d'un cycle pyrrolidine ou perhydroazépine comme c'est le cas pour les dérivés de la présente invention.

Plus spécifiquement, la présente invention concerne des composés de formule (I) : 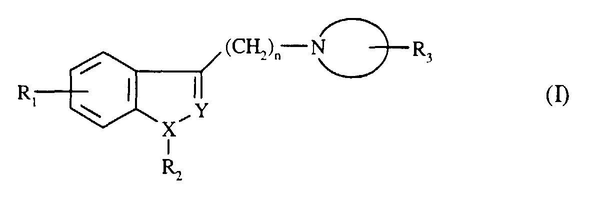 dans laquelle :
- R₁: représente un atome d'hydrogène, d'halogène, un groupement alkyle (C₁-C₆) linéaire ou ramifié, alkoxy (C₁-C₆) linéaire ou ramifié, trihalogénométhyle ou hydroxy,
- R₂: représente un atome d'hydrogène, un groupement alkyle (C₁-C₆) linéaire ou ramifié, phényle (substitué ou non par un ou plusieurs atomes d'halogène ou groupement alkyle, alkoxy, hydroxy ou trihalogénométhyle), 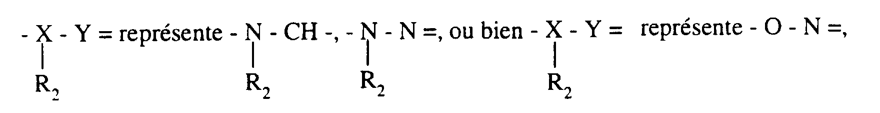 1≤ n ≤ 6, 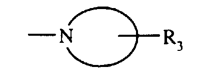 représente l'un quelconque des groupements suivants : 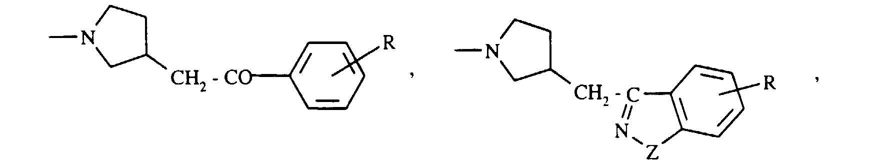 ou 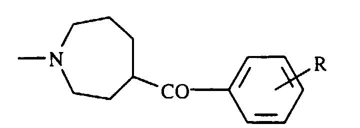 dans lesquelles :
- R: représente un atome d'hydrogène, d'halogène, un groupement alkyle (C₁-C₆) linéaire ou ramifié, alkoxy (C₁-C₆) linéaire ou ramifié, hydroxy ou trihalogénométhyle,
- Z: représente un atome d'oxygène, de soufre ou un groupement -NH-,
leurs isomères ainsi que leurs sels d'addition à un acide ou à une base pharmaceutiquement acceptable.

Par isomère, on entend les énantiomères, diastéréoisomères et épimères ainsi que les isomères de conformation.

Parmi les acides pharmaceutiquement acceptables, on peut citer à titre non limitatif les acides chlorhydrique, bromhydrique, sulfurique, phosphorique, acétique, trifluoroacétique, lactique, pyruvique, malonique, succinique, glutarique, fumarique, tartrique, maléïque, citrique, ascorbique, oxalique, méthane sulfonique, camphorique, etc...

Parmi les bases pharmaceutiquement acceptables, on peut citer à titre non limitatif l'hydroxyde de sodium, l'hydroxyde de potassium, la triéthylamine, la tertbutylamine, etc...

L'invention s'étend également au procédé de préparation des composés de formule (I) caractérisé en ce que l'on utilise comme produit de départ un alcool de formule (II) : 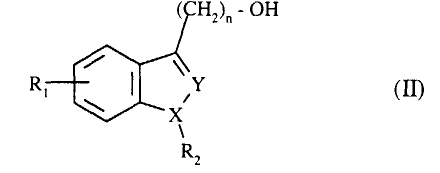 dans laquelle R₁, R₂, X, Y et n ont la même signification que dans la formule (I),
que l'on transforme en dérivé bromé correspondant à l'aide de tétrabromure de carbone en présence de triphénylphosphine,
pour conduire au composé de formule (III) : 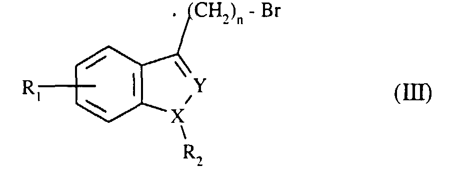 dans laquelle R₁, R₂, X, Y et n ont la même signification que dans la formule (I),
que l'on fait réagir sur une amine de formule (IV) : 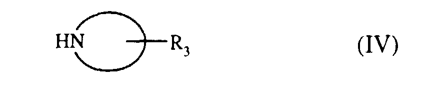 dans laquelle R₃ a la même signification que dans la formule (I),
pour conduire au composé de formule (I),
- qui peut être, le cas échéant, purifié selon une technique classique de purification
- dont on sépare, le cas échéant, les isomères selon une technique classique de séparation,
- que l'on transforme, si on le souhaite, en ses sels d'addition à une base pharmaceutique acceptable.

Les composés de formule (II) sont obtenus :
- lorsque n est égal à 1 par réduction des acides ou des aldéhydes correspondants par de l'hydrure de lithium aluminium;
- lorsque n est égal à 2 ou 3 par réaction d'une phénylhydrazine correctement substituée avec le 2-éthoxytétrahydrofurane (n=2) ou le 2-méthoxytétrahydropyrane (n=3) selon la méthode décrite dans le brevet BE 892145 ;
- lorsque n > 3 par transformation des dérivés bromés de formule (III) en nitriles correspondants, puis par alcoolyse en esters correspondants, puis réduction en alcool et ainsi de suite jusqu'à obtention de la longueur de chaîne souhaitée.

Les composés de formule (II) dans laquelle 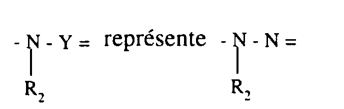 sont obtenus plus spécifiquement selon le procédé décrit dans le brevet JP 8031050.

Les composés de formule (IV) tels que 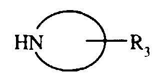 représente le groupement : 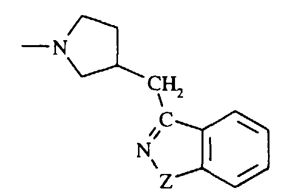 sont obtenus selon le procédé décrit dans le brevet EP 061034.

La présente invention a également pour objet les compositions pharmaceutiques renfermant comme principe actif au moins un composé de formule (I) seul ou en combinaison avec un ou plusieurs excipients ou véhicules inertes non toxiques.

Parmi les compositions pharmaceutiques selon l'invention, on pourra citer plus particulièrement celles qui conviennent pour l'administration orale, parentérale, nasale, les comprimés simples ou dragéifiés, les comprimés sublinguaux, les gélules, les tablettes, les suppositoires, les crèmes, pommades, gels dermiques, etc...

La posologie utile varie selon l'âge et le poids du patient, la nature et la sévérité de l'affection ainsi que la voie d'administration. Celle-ci peut être orale, nasale, rectale ou parentérale. D'une manière générale, la posologie unitaire s'échelonne entre 10 µg et 100 mg pour un traitement en 1 à 3 prises par 24 heures.

Les exemples suivants illustrent l'invention et ne la limitent en aucune façon.

Les produits de départ utilisés sont des produits connus ou préparés selon des modes opératoires connus.

### EXEMPLE 1:

### 3-{2-[3-(4-Fluorobenzoylméthyl)pyrrolidin-1-yl]éthyl}-5-méthoxy-indole, chlorhydrate

### Stade 1:

### 2-(5-Méthoxy-indol-3-yl)éthanol

Ajouter une solution de 15 g d'acide (5-méthoxy-indol-3-yl)acétique (0,073 mol) dans 100 ml de tétrahydrofurane à une suspension de 2,1 g (0,219 mol) d'hydrure de lithium-aluminium dans 100 ml d'éther éthylique et porter 6 heures à reflux. Hydrolyser ensuite le mélange réactionnel par 80 ml d'une solution aqueuse saturée de sulfate de magnésium. Filtrer l'ensemble sur célite et évaporer les solvants sous vide. Le résidu est repris dans un peu d'eau et extrait plusieurs fois à l'aide de dichlorométhane. Après évaporation du solvant, on obtient le produit attendu.

### Stade 2 :

### 3-(2-Bromoéthyl)-5-méthoxy-indole

Laisser agiter 2 heures un mélange de 10 g du produit obtenu au stade 1 (0,0523 mol), 16,5 g de triphénylphosphine (0,0628 mol), 17,3 g de tétrabromure de carbone (0,0628 mol) et 200 ml d'acétonitrile. Evaporer ensuite le solvant sous vide et purifier le produit par chromatographie sur colonne de silice Merck 70-230 Mesh en utilisant du dichlorométhane comme éluant.

### Stade 3 :

### 3-{2-[3-(4-Fluorobenzoylméthyl)pyrrolidin-1-yl[éthyl}-5-méthoxy-indole

Porter à reflux pendant 24 heures un mélange de 5 g (0,0197 mol) du produit obtenu au stade 2, 4 g (0,0216 mol) de 3-(4-fluorobenzoylméthyl)-pyrrolidine, 0,1 g d'iodure de sodium et 3 g de carbonate de potassium dans 100 ml de diéthylcétone. Filtrer ensuite les sels minéraux, évaporer le solvant sous vide, reprendre le résidu dans 100 ml de dichlorométhane et laver cette phase organique avec 100 ml d'eau. Après séchage sur sulfate de magnésium et évaporation du solvant, le résidu est purifié par chromatographie sur colone de silice Merck 70-230 Mesh en utilisant comme éluant un mélange dichlorométhane/méthanol/ammoniaque (97/3/0,3).

### Stade 4 :

### 3-{2-[3-(4-Fluorobenzoylméthyl)pyrrolidin-1-yl]éthyl}-5-méthoxy-indole, chlorhydrate

Ajouter un équivalent de chlorure d'hydrogène en solution dans l'éthanol à une solution acétonique du produit obtenu au stade 3. L'évaporation totale des solvants conduit au chlorhydrate du composé cité.

| *Microanalyse élémentaire :* | | | | |
|---|---|---|---|---|
| | *C %* | *H %* | *N %* | *Cl %* |
| *calculé* | *66,26* | *6,29* | *6,72* | *8,50* |
| *trouvé* | *66,53* | *6,30* | *6,38* | *8,13* |

### EXEMPLE 2 :

### 1-Méthyl-3-{2-[3-(4-fluorobenzoylméthyl)pyrrolidin-1-yl]éthyl}-5-méthoxy-indole, chlorhydrate

### Stade 1:

### 2-(5-Méthoxy-1-méthyl-indol-3-yl)-acétate de méthyle

A une solution de 10 g (0,0487 mol) d'acide (5-méthoxy-indol-3-yl)acétique dans 120 ml d'acétone, ajouter 8,2 g (0,1464 mol) d'hydroxyde de potassium en poudre et 13,8 g (0,0972 mol) d'iodure de méthyle. Après 4 heures d'agitation à température ambiante, ajouter à nouveau 13,8 g d'iodure de méthyle, puis 12 heures plus tard 13,8 g d'iodure de méthyle et 8 g d'hydroxyde de potassium. Puis concentrer le tout, reprendre dans l'eau le résidu, extraire à l'aide de dichlorométhane, laver la phase organique par une solution de soude 1N puis à l'aide d'acide chlorhydrique 1N, décanter, sécher la solution organique sur sulfate de magnésium et évaporer le solvant sous vide.

### Stade 2 :

### 2-(5-Méthoxy-1-méthyl-indol-3-yl)-éthanol

La réduction de l'ester obtenu au stade 1 s'effectue selon le mode opératoire décrit au stade 1 de l'exemple 1.

### Stade 3 :

### 3-(2-Bromoéthyl)-5-méthoxy-1-méthyl-indole

La bromation de l'alcool obtenu au stade 2 se réalise de la même manière que celle décrite au stade 2 de l'exemple 1.

| *Microanalyse élémentaire :* | | | | |
|---|---|---|---|---|
| | *C %* | *H %* | *N %* | *Br %* |
| *calculé* | *53,75* | *5,26* | *5,22* | *29,8* |
| *trouvé* | *53,60* | *5,21* | *5,20* | *29,7* |

### Stade 4 :

### 1-Méthyl-3-{2-[3-(4-fluorobenzoylméthyl)pyrrolidin-1-yl]éthyl}-5-méthoxy-indole

L'alkylation de la 3-(4-fluorobenzoylméthyl)-pyrrolidine par le composé bromé obtenu au stade 3 se réalise dans la diéthylcétone en suivant le mode opératoire décrit au stade 3 de l'exemple 1.

### Stade 5 :

### 1-Méthyl-3-{2-[3-(4-fluorobenzoylméthyl)pyrrolidin-1-yl]éthyl}-5-méthoxy-indole, chlorhydrate

La salification du composé obtenu au stade 4 de l'exemple 2 s'effectue de manière semblable à celle décrite au stade 4 de l'exemple 1.

| *Microanalyse élémentaire :* | | | | |
|---|---|---|---|---|
| | *C %* | *H %* | *N %* | *Cl %* |
| *calculé* | *66,89* | *6,55* | *6,50* | *8,24* |
| *trouvé* | *65,22* | *6,43* | *6,29* | *8,15* |

### EXEMPLE 3 :

### 3-{3-[3-(4-Fluorobenzoylméthyl)-pyrrolidin-1-yl]propyl}-5-méthoxy-indole, chlorhydrate

### Stade 1:

### 3-(5-Méthoxy-indol-3-yl)-propan-1-ol

Cet alcool est synthétisé selon la méthode décrite dans le brevet belge BE 892145 en utilisant le chlorhydrate de 4-méthoxy-phénylhydrazine et le 2-méthoxy-tétrahydropyrane comme matières premières.

### Stade 2 :

### 3-(3-Bromopropyl)-5-méthoxy-indole

Ce composé est synthétisé selon la méthode décrite au stade 2 de l'exemple 1 en utilisant le composé du stade 1 de l'exemple 3 comme matière première.

### Stade 3 :

### 3-{3-[3-(4-Fluorobenzoylméthyl)-pyrrolidin-1-yl]propyl}-5-méthoxy-indole

Ce composé est synthétisé selon la méthode décrite au stade 3 de l'exemple 1 à partir du composé décrit au stade précédent.

### Stade 4:

### 3-{3-[3-(4-Fluorobenzoylméthyl)-pyrrolidin-1-yl]propyl}-5-méthoxy-indole, chlorhydrate

La salification est réalisée de la même manière que celle décrite au stade 4 de l'exemple 1.

| *Microanalyse élémentaire :* | | | | |
|---|---|---|---|---|
| | *C %* | *H %* | *N %* | *Cl %* |
| *calculé* | *66,89* | *6,55* | *6,50* | *8,23* |
| *trouvé* | *66,53* | *6,47* | *6,24* | *8,00* |

### EXEMPLES 4 ET 5 :

### Isomères α et β du chlorhydrate de 3-{3-[3-(4-fluorobenzoylméthyl)-pyrrolidin-1-yl]propyl}-5-méthoxy-indole

La synthèse des énantiomères du composé de l'exemple 3 (racémique) s'effectue en utilisant les énantiomères purs de la 3-(4-fluorobenzoylméthyl)-pyrrolidine dont la préparation a été décrite dans le brevet EP 389352.

*Rendement : 45* %

### EXEMPLE 4 : isomère α

| *Microanalyse élémentaire :* | | | | |
|---|---|---|---|---|
| | *C %* | *H %* | *N %* | *Cl %* |
| *calculé* | *66,89* | *6,55* | *6,50* | *8,23* |
| *trouvé* | *67,00* | *6,56* | *6,07* | *8,27* |

*Pouvoir rotatoire :* [α]_{D}²¹ = - 1,85° (c = 1 %, DMSO)
*Pureté énantiomère :* ≥ *99* % *(colonne chirale)*

### EXEMPLE 5 : isomère β

| *Microanalyse élémentaire :* | | | | |
|---|---|---|---|---|
| | *C %* | *H %* | *N %* | *Cl %* |
| *calculé* | *66,89* | *6,55* | *6,50* | *8,23* |
| *trouvé* | *66,90* | *6,55* | *6,65* | *8,19* |

*Pouvoir rotatoire :* [α]_{D}²¹ = + 1,40° (c = 1 %, DMSO)
*Pureté énantiomère :* ≥ 99 % *(colonne chirale)*

### EXEMPLE 6 :

### 1-Méthyl-3-{3-[3-(4-fluorobenzoylméthyl)-pyrrolidin-1-yl]propyl}-5-méthoxy-indole, chlorhydrate

### Stade 1 :

### 3-(5-Méthoxy-1-méthyl-indol-3-yl)-propan-1-ol

Le produit attendu est obtenu par méthylation du 3-(5-méthoxy-indol-3-yl)-propan-1-ol décrit au stade 1 de l'exemple 3 en utilisant la technique décrite au stade 1 de l'exemple 2.

| *Microanalyse élémentaire :* | | | |
|---|---|---|---|
| | *C %* | *H %* | *N %* |
| *calculé* | *71,21* | *7,81* | *6,39* |
| *trouvé* | *71,37* | *7,56* | *6,20* |

### Stade 2 :

### 3-(3-Bromopropyl)-5-méthoxy-1-méthyl-indole

La synthèse du dérivé bromé est réalisée selon la méthode décrite au stade 2 de l'exemple 1.

### Stade 3 :

### 1-Méthyl-3-{3-[3-(4-fluorobenzoylméthyl)-pyrrolidin-1-yl]propyl}-5-méthoxy-indole

L'alkylation de la 3-(4-fluorobenzoylméthyl)-pyrrolidine par le dérivé bromé obtenu au stade précédent s'effectue selon le mode opératoire décrit au stade 4 de l'exemple2.

### Stade 4:

### 1-Méthyl-3-{3-[3-(4-fluorobenzoylméthyl)-pyrrolidin-1-yl]propyl}-5-méthoxy-indole, chlorhydrate

| *Microanalyse élémentaire :* | | | | |
|---|---|---|---|---|
| | *C*% | *H%* | *N%* | *Cl%* |
| *calculé* | *67,48* | *6,80* | *6,30* | *7,97* |
| *trouvé* | *67,24* | *6,70* | *6,24* | *8,09* |

### EXEMPLE 7:

### 3-{3-[4-(4-Fluorobenzoyl)perhydroazépin-1-yl]propyl}-5-méthoxy-indole, chlorydrate

Ce composé est obtenu selon le procédé décrit dans l'exemple 1 en utilisant au stade 3 la 4-(4-fluorobenzoyl)azépine décrite dans le brevet EP 389352 à la place de la 3-(4-fluorobenzoylméthyl)pyrrolidine.

*Rendement : 60* %
*Point de fusion : 139-140°C*

| *Microanalyse élémentaire :* | | | | |
|---|---|---|---|---|
| | *C %* | *H %* | *N %* | *Cl %* |
| *calculé* | *67,48* | *6,80* | *6,30* | *7,97* |
| *trouvé* | *67,22* | *6,68* | *6,13* | *7,79* |

### EXEMPLE 8:

### 3-{3-[3-(4-Fluorobenzoylméthyl)pyrrolidin-1-yl]propyl}-5-fluoroindole, chlorhydrate

Ce composé est obtenu selon le procédé décrit dans l'exemple 3 en utilisant comme matière première au stade 1 le chlorhydrate de 4-fluorophényl hydrazine à la place du chlorhydrate de 4-méthoxyphényl hydrazine.

| *Microanalyse élémentaire :* | | | | |
|---|---|---|---|---|
| | *C %* | *H %* | *N %* | *Cl %* |
| *calculé* | *65,95* | *6,02* | *6,69* | *8,46* |
| *trouvé* | *65,98* | *6,25* | *6,23* | *8,51* |

### EXEMPLE 9 :

### 6-Fluoro-3-{1-[3-(5-méthoxy-indol-3-yl)propyl]pyrrolidin-3-yl-méthyl}1,2-benzisoxazole, chlorhydrate

Ce composé est obtenu selon le procédé décrit dans l'exemple 3 en remplacant au stade 3 la 3-(4-fluorobenzoylméthyl)pyrrolidine par le 3-[(pyrrolidin-3-yl)méthyl]-6-fluoro- 1,2-benzisoxazole obtenu selon le procédé décrit dans le brevet EP 0610134.

| *Microanalyse élémentaire :* | | | | |
|---|---|---|---|---|
| | *C %* | *H %* | *N %* | *Cl %* |
| *calculé* | *64,93* | *6,13* | *9,46* | *7,99* |
| *trouvé* | *63,31* | *6,08* | *8,92* | *8,04* |

### EXEMPLE 10:

### 3-{2-[3-(4-Fluorobenzoylméthyl)pyrrolidin-1-yl]éthyl}-5-chloro-1H-indazole, chlorhydrate

### Stade 1:

### Acide 3-amino-3-(5-chloro-2-nitrophényl)propanoïque

Dans un tricol muni d'un thermomètre, d'un agitateur mécanique et d'un réfrigérant surmonté d'une garde à silicagel, un mélange de 50 g de 5-chloro-2-nitrobenzaldéhyde (0,27 mole), de 4 eq d'acide formique (49,6 g) et de 1,3 eq d'acide malonique (36,6 g) est porté à 40-45°C. On ajoute 2,5 eq de formiate d'ammonium. On chauffe 1 heure à 60-70°C puis 5 heures à 90-95°C. On procède ensuite à l'acidification en ajoutant 9,8 eq d'acide chlorhydrique à 36 % (110 ml) et 20 ml d'eau poursuivant le reflux pendant une heure. On concentre à sec, puis on resolubilise dans de l'eau. On ramène le pH vers 7,8 avec de la soude 2M. Le précipité obtenu est filtré puis séché au dessicateur. On obtient l'amino-acide sous la forme de sel interne.

### Stade 2 :

### Acide (5-chloro-1H-indazol-3-yl)acétique

Dans un tricol muni d'un thermomètre, d'un agitateur mécanique, d'une ampoule de coulée et d'un réfrigérant surmonté d'un bulleur, on solubilise l'amino-acide obtenu au stade 1 avec 3 équivalents de soude en pastille dans 600 ml d'eau. Le mélange est porté à 30-40°C, on ajoute 5,7 g de charbon actif. Le mono-hydrate d'hydrazine est additionné lentement (2,1 eq soit 22,7 g). Le mélange est agité 15 minutes à 30-40°C puis 3 heures à 80-85°C. Le milieu réactionnel est refroidi. Après filtration le milieu réactionnel est acidifié avec 75 ml d'acide chlorhydrique à 36 %. Le précipité formé est filtré sur fritté puis séché au dessicateur.
*Point de fusion : 193°C*

### Stade 3 :

### 2-(5-Chloro-1H-indazol-3-yl)éthanol

Dans un tricol équipé d'un thermomètre, d'une ampoule de coulée et d'un réfrigérant surmonté d'un bulleur relié à une garde de silicagel, on introduit 6,7 g (0,95 eq) d'hydrure de lithium aluminium que l'on recouvre de 80 ml de tétrahydrofurane. L'acide obtenu au stade précédent solubilisé dans 420 ml de tétrahydrofurane est additionné lentement sur l'hydrure. Le reflux est maintenu pendant 24 heures avec agitation. Le milieu réactionnel refroidi à température ambiante est hydrolysé avec 180 ml de solution de sulfate de magnésium saturée (10 eq). Il se forme un précipité que l'on filtre sur célite. Le filtrat est concentré à sec. Le résidu est repris dans de l'acétate d'éthyle avec un peu d'eau. La phase organique est de nouveau filtrée pour éliminer les sels minéraux, le filtrat est concentré. Le produit est purifié par chromatographie sur colonne de silice en utilisant comme éluant l'acétate d'éthyle.

### Stade 4 :

### 3-(2-Bromoéthyl)-5-chloro-1 H-indazole

Le produit attendu est obtenu selon le procédé décrit au stade 2 de l'exemple 1 à partir du composé obtenu au stade précédent.

### Stade 5 :

### 3-{2-[3-(4-Fluorobenzoylméthyl)pyrrolidin-1-yl]éthyl}-5-chloro-1H-indazole

Le produit attendu est obtenu selon le procédé décrit au stade 3 de l'exemple 1 à partir du composé décrit au stade précédent.

### Stade 6:3-{2-[3-(4-Fluorobenzoylméthyl)pyrrolidin-1-yl]éthyl}-5-chloro-1H-indazole, chlorhydrate

Le chlorhydrate du produit obtenu au stade précédent est obtenu selon le procédé décrit au stade 4 de l'exemple 1.

### Etude pharmacologique des composés de l'invention

Les composés de l'invention ont été testés comparativement à l'halopéridol, à la clozapine et à un composé nommé Ref. A qui possède un cycle pipéridine à la place d'un cycle pyrrolidine ou perhydroazépine comme c'est le cas pour les composés de l'invention.
Ref. A:3-{3-[4-(4-Fluorobenzoyl)pipéridin-1-yl]propyl}-5-méthoxy-indole, chlorhydrate.

### EXEMPLE 11 :

### Profil réceptoriel des composés de l'invention

L'interaction de ces composés avec différents récepteurs a été déterminée en utilisant des études de fixation classiques comme celles décrites par M.J. MILLAN et coll. (J. Pharmacol. Exp. Ther., 268, 337-352, 1994 et Drug News Perspectives, 5, 397-406, 1992). Le profil réceptoriel des composés de l'invention est présenté dans le tableau ci-dessous. Les affinités des composés sont exprimées en Ki.

| Profil réceptoriel (Ki, nM) | | | | | | | |
|---|---|---|---|---|---|---|---|
| **Exemples** | **D**_{**2**} | **5-HT**_{**2A**} | **5-HT**_{**2C**} | **5-HT**_{**1A**} | **D**_{**2**}**/5-HT**_{**2A**} | **D**_{**2**}**/5-HT**_{**2C**} | **D**_{**2**}**/5-HT**_{**1A**} |
| Halopéridol | 1 | 83 | 5754 | 2818 | 0.01 | <0.01 | <0.01 |
| Clozapine | 186 | 23 | 8.7 | 295 | 8.11 | 21 | 0.6 |
| Ref. A | 11 | 8.5 | 98 | 5.6 | 1.3 | 0.1 | 2 |
| Ex. 1 | 110 | 1.6 | 50 | 0.6 | 69 | 22 | 183 |
| Ex. 2 | 132 | 1.3 | 16 | 141 | 101 | 8.3 | 0.9 |
| Ex. 3 | 5.2 | 1.0 | 3.3 | 1.0 | 5.2 | 1.6 | 5.2 |
| Ex. 4 | 6.3 | 1.20 | 4.0 | 1.1 | 5.2 | 1.6 | 5.7 |
| Ex. 5 | 7.1 | 1.7 | 3.8 | 0.4 | 4.2 | 1.9 | 17.8 |
| Ex. 6 | 15.4 | 4.3 | 17 | 39 | 3.6 | 0.9 | 0.4 |
| Ex. 7 | 43.5 | 2.7 | 117 | 0.7 | 1.6 | 0.4 | 62 |
| Ex. 8 | 9.5 | 6.6 | 19.0 | 12.3 | 1.4 | 0.5 | 0.8 |
| Ex. 9 | 98 | 3.2 | 7.9 | 0.7 | 31 | 13 | 143 |

Ces résultats montrent la forte affinité des composés de l'invention pour les récepteurs 5-HT _{1A}, 5-HT_{2A} et plus particulièrement 5-HT_{2C} par rapport aux récepteurs D₂ à l'instar de la clozapine et à la différence de l'halopéridol et du composé Ref. A.

### EXEMPLE 12:

### Propriétés antipsychotiques

Afin de déterminer les propriétés antipsychotiques des composés de l'invention, un test bien établi a été utilisé dans lequel l'ensemble des antipsychotiques sont cliniquement actifs comme l'ont montré A.Y. DEUTCH et coll. (Schizophrenia Research, 4, 121-156, 1991) : inhibition de la verticalisation induite par un agoniste dopaminergique : l'apomorphine (selon le protocole décrit par P. PROTAIS et coll., Psychopharmacol., 50, 1-6, 1976).
Une activité dans ces tests semble refléter un blocage des voies dopaminergiques mésolimbiques dont une hyperactivité est supposée chez les schizophrènes comme décrit par P.C. WALDMEIER et coll. (Eur. J. Pharmacol., 55, 363-373, 1979) et A.Y. DEUTCH cité plus haut. Les activités des composés de l'invention ont été comparées à celles de l'halopéridol et de la clozapine.

### Test de verticalisation à l'apomorphine chez la souris

L'expérience a été effectuée sur des souris CF (Charles River) mâles de poids moyen 25 g. Trente minutes avant le début du test, chaque souris a été placée dans une cage cylindrique (Ø 12 cm x 14 cm), à barreaux verticaux métalliques et couvercle plastique lisse, après avoir reçu une injection sous-cutanée de solvant ou de produit. A T₀, une solution d'apomorphine (0,75 mg/kg) ou de sérum physiologique a été administrée par voie sous-cutanée à l'animal, placé à nouveau dans la cage à barreaux. A T₁₀ (10 minutes) et à T₂₀ (20 minutes), un score a été attribué à chaque animal après une minute d'observation environ :
- score 0 (4 pattes au sol) ;
- score 1 (animal redressé, pattes avant sur les barreaux verticaux) ;
- score 2 (animal accroché par les 4 pattes sur les barreaux).

Le score total des 2 mesures représente alors la valeur de verticalisation de l'animal, utilisée pour l'analyse statistique.

### EXEMPLE 13:

### Catalepsie chez le rat

Afin de déterminer le potentiel des composés de l'invention à engendrer un syndrome de type extrapyramidal chez l'homme, la capacité à induire une catalepsie chez le rat a été examinée. Ce phénomène est dû à un antagonisme de la transmission dopaminergique nigrostriatale. Les activités des composés ont été comparées à celles de l'halopéridol et la clozapine.

### Protocole :

Les rats Wistar, mâles (220-240 g) ont été placés en cages individuelles et mis à jeun la veille du test. Le test utilisé pour évaluer les propriétés cataleptogènes d'un produit consiste à placer chaque patte arrière de l'animal sur la patte avant située du même côté et à mesurer le temps en secondes pendant lequel l'animal a gardé cette position de "pattes croisées", jusqu'à 30 secondes comme décrit par P.C. WALDMEIER et coll. (Eur. J. Pharmacol., 55, 363-373, 1979). Chaque animal a subi trois essais, à une minute d'intervalle ; la valeur moyenne des trois essais représente alors le temps de catalepsie de l'animal utilisé pour l'analyse statistique.
Le produit à tester a été administré à l'animal, par voie sous-cutanée, 30 minutes avant le test.
Les résultats de l'induction de la catalepsie chez le rat sont présentés dans le tableau ci-dessous :
La dose cataleptogène efficace DE₅₀ est celle qui induit une catalepsie d'une durée moyenne de 15 secondes, c'est-à-dire 50 % de la durée maximale du test (30 secondes).
Les résultats des tests décrits dans les exemples 12 et 13 sont présentés dans le tableau ci-dessous :

| **Exemples** | **Verticalisation (Apomorphine) ID**_{**50**} **(mg/kg/S.C.)** | **Induction de la catalepsie ED**_{**50**} **(mg/kg/S.C.)** | **Rapport ED**_{**50**}**/ID**_{**50**} |
|---|---|---|---|
| Halopéridol | 0.01 | 0.14 | 14 |
| Clozapine | 2.2 | > 40.0 | > 18 |
| Ref. A | 0.1 | 1.4 | 14 |
| Ex. 1 | 0.31 | > 40.0 | > 129 |
| Ex. 2 | 1.25 | > 40.0 | > 32 |
| Ex. 3 | 0.15 | > 40.0 | > 266 |
| Ex. 4 | 0.3 | > 40.0 | > 133 |
| Ex. 5 | 0.13 | > 40.0 | > 307 |
| Ex. 7 | 0.5 | > 40.0 | > 80 |
| Ex. 8 | 0.5 | > 40.0 | > 80 |
| Ex. 9 | 1.0 | > 40.0 | > 40 |

Ces résultats montrent la forte activité antipsychotique inhibitrice de la verticalisation des composés de l'invention en l'absence des propriétés extrapyramidales (induction de la catalepsie) à l'instar de la clozapine et à la différence de l'halopéridol et du composé Ref. A.

### EXEMPLE 14:

### Composition pharmaceutique

| Formule de préparation pour 1000 comprimés dosés à 1 mg | |
|---|---|
| Composé de l'exemple 2 | 1 g |
| Hydroxypropylcellulose | 2 g |
| Amidon de blé | 10 g |
| Lactose | 100 g |
| Stéarate de magnésium | 3 g |
| Talc | 3 g |

## Revendications

1. Composé de formule (I) : 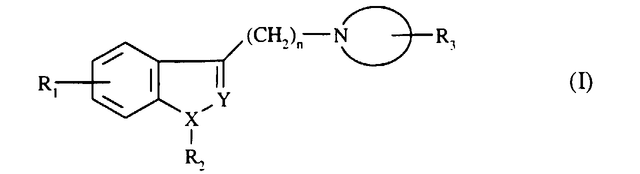 dans laquelle :
R₁ représente un atome d'hydrogène, d'halogène, un groupement alkyle (C₁-C₆) linéaire ou ramifié, alkoxy (C₁-C₆) linéaire ou ramifié, trihalogénométhyle ou hydroxy,
R₂ représente un atome d'hydrogène, un groupement alkyle (C₁-C₆) linéaire ou ramifié, phényle (substitué ou non par un ou plusieurs atomes d'halogène ou groupement alkyle, alkoxy, hydroxy ou trihalogénométhyle), 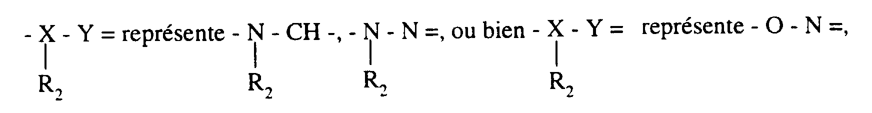 1 ≤ n ≤ 6, 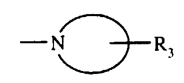 représente l'un quelconque des groupements suivants : 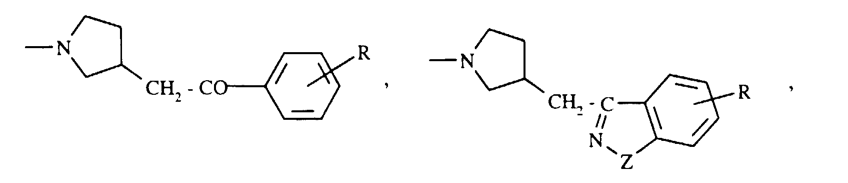 ou 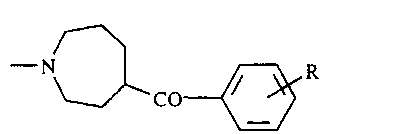 dans lesquelles :
R représente un atome d'hydrogène, d'halogène, un groupement alkyle (C₁-C₆) linéaire ou ramifié, alkoxy (C₁-C₆) linéaire ou ramifié, hydroxy ou trihalogénométhyle,
Z représente un atome d'oxygène, de soufre ou un groupement -NH-,
leurs isomères ainsi que leurs sels d'addition à un acide ou à une base pharmaceutiquement acceptable.

2. Composé de formule (I) selon la revendication 1 caractérisé en ce que 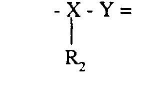 représente - NH - CH =

3. Composé de formule (I) selon la revendication 1 caractérisé en ce que n est égal à 2.

4. Composé de formule (I) selon la revendication 1 caractérisé en ce que n est égal à 3.

5. Composé de formule (I) selon la revendication 1 caractérisé en ce que 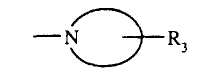 représente

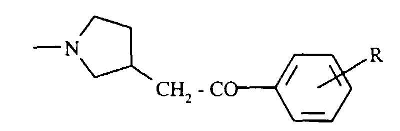

6. Composé de formule (I) selon la revendication 1 caractérisé en ce que 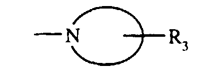 représente

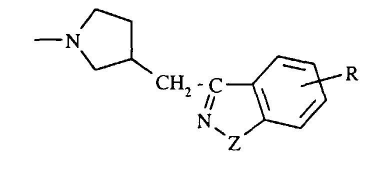

7. Composé de formule (I) selon la revendication 1 caractérisé en ce que 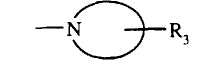 représente

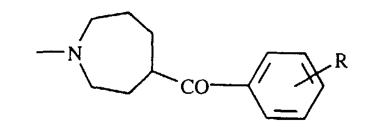

8. Procédé de préparation des composés de formule (I) selon la revendication 1 caractérisé en ce que l'on utilise comme produit de départ un alcool de formule (II) : 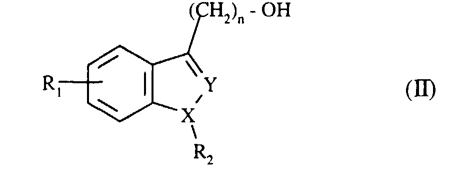 dans laquelle R₁, R₂, X, Y et n ont la même signification que dans la formule (I),
que l'on transforme en dérivé bromé correspondant à l'aide de tétrabromure de carbone en présence de triphénylphosphine,
pour conduire au composé de formule (III) : 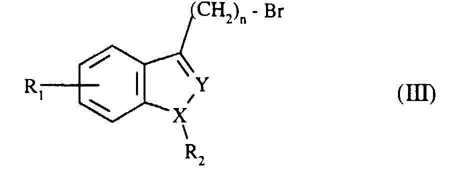 dans laquelle R₁, R₂, X, Y et n ont la même signification que dans la formule (I),
que l'on fait réagir sur une amine de formule (IV) : 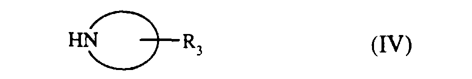 dans laquelle R₃ a la même signification que dans la formule (I),
pour conduire au composé de formule (I),
- qui peut être, le cas échéant, purifié selon une technique classique de purification
- dont on sépare, le cas échéant, les isomères selon une technique classique de séparation,
- que l'on transforme, si on le souhaite, en ses sels d'addition à une base pharmaceutique acceptable.

9. Composition pharmaceutique contenant comme principe actif au moins un composé selon l'une quelconque des revendications 1 à 7, seul ou en combinaison avec un ou plusieurs véhicules inertes, non toxiques, pharmaceutiquement acceptable.

10. Composition pharmaceutique selon la revendication 9 contenant au moins un principe actif selon l'une des revendications 1 à 7 utile dans le traitement de la schizophrénie.

## Patentansprüche

1. Verbindung der Formel (I): 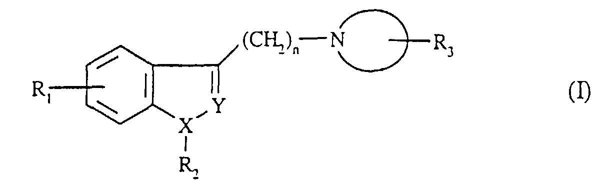 in der:
R₁ ein Wasserstoffatom, ein Halogenatom, eine geradkettige oder verzweigte (C₁-C₆)-Alkylgruppe, eine geradkettige oder verzweigte (C₁-C₆)-Alkoxygruppe, eine Trihalogenmethylgruppe oder eine Hydroxygruppe,
R₂ ein Wasserstoffatom, eine geradkettige oder verzweigte (C₁-C₆)-Alkylgruppe, eine Phenylgruppe (die gegebenenfalls durch ein oder mehrere Halogenatome oder Alkyl-, Alkoxy-, Hydroxy- oder Trihalogenmethylgruppen substituiert ist), 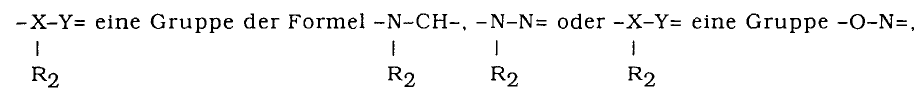 1 ≤ n ≤ 6 und 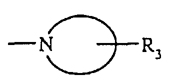 eine der folgenden Gruppen: 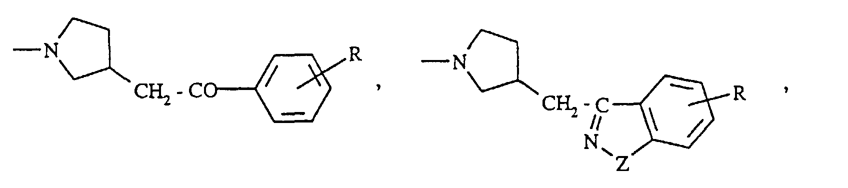 oder 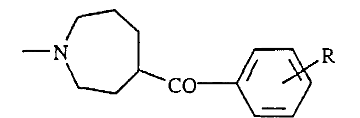 worin
R ein Wasserstoffatom, ein Halogenatom, eine geradkettige oder verzweigte (C₁-C₆)-Alkylgruppe, eine geradkettige oder verzweigte (C₁-C₆)-Alkoxygruppe, eine Hydroxygruppe oder eine Trihalogenmethylgruppe und
Z ein Sauerstoffatom, ein Schwefelatom oder eine Gruppe -NH- darstellen, bedeuten, deren Isomere sowie deren Additionssalze mit einer pharmazeutisch annehmbaren Säure oder Base.

2. Verbindung der Formel (I) nach Anspruch 1, dadurch gekennzeichnet, daß 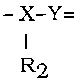 eine Gruppe der Formel -NH-CH= darstellt.

3. Verbindung der Formel (I) nach Anspruch 1, dadurch gekennzeichnet, daß n den Wert 2 besitzt.

4. Verbindung der Formel (I) nach Anspruch 1, dadurch gekennzeichnet, daß n den Wert 3 besitzt.

5. Verbindung der Formel (I) nach Anspruch 1, dadurch gekennzeichnet, daß 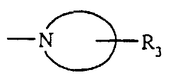 für die Formel 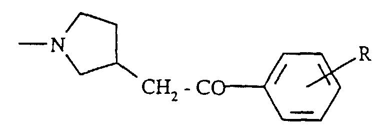 steht.

6. Verbindung der Formel (I) nach Anspruch 1, dadurch gekennzeichnet, daß 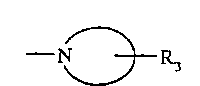 für die Formel 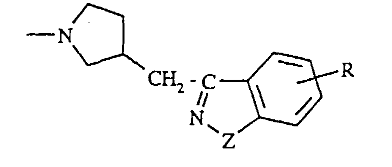 steht.

7. Verbindung der Formel (I) nach Anspruch 1, dadurch gekennzeichnet, daß 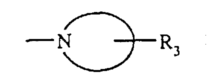 für die Formel 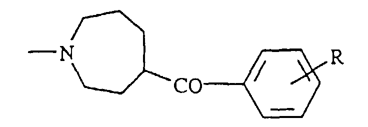 steht.

8. Verfahren zur Herstellung der Verbindungen der Formel (I) nach Anspruch 1, dadurch gekennzeichnet, daß man als Ausgangsprodukt einen Alkohol der Formel (II) verwendet: 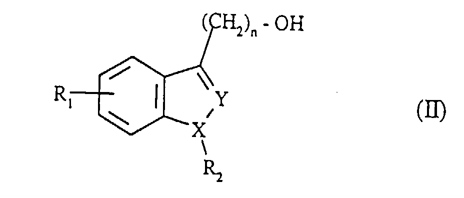 in der R₁, R₂, X, Y und n die bezüglich der Formel (I) angegebenen Bedeutungen besitzen,
welche man mit Hilfe von mit Tetrabrommethan in Gegenwart von Triphenylphosphin in das Bromderivat umwandelt zur Bildung der Verbindung der Formel (III): 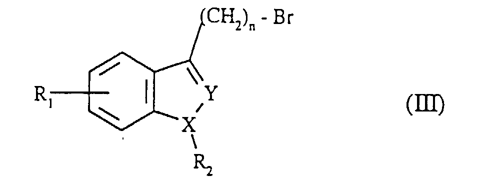 in der R₁, R₂, X, Y und n die bezüglich der Formel (I) angegebenen Bedeutungen besitzen,
welche man mit einem Amin der Formle (IV): 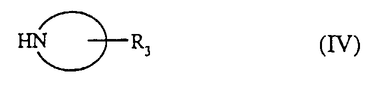 in der R₃ die bezüglich der Formel (I) angegebenen Bedeutungen besitzt, umsetzt zur Bildung der Verbindung der Formel (I),
- welche gegebenenfalls mit Hilfe einer klassischen Reinigungsmethode gereinigt werden kann,
- welche man gegebenenfalls mit Hilfe einer klassischen Trennmethode in die Isomeren auftrennen kann, und
- welche man gewünschtenfalls in ihre Additionssalze mit einer pharmazeutisch annehmbaren Base umwandeln kann.

9. Pharmazeutische Zubereitung enthaltend als Wirkstoff mindestens eine Verbindung nach einem der Ansprüche 1 bis 7 allein oder in Kombination mit einem oder mehreren inerten, nichttoxischen, pharmazeutisch annehmbaren Trägermaterialien.

10. Pharmazeutische Zubereitung nach Anspruch 9 enthaltend mindestens einen Wirkstoff gemäß einem der Ansprüche 1 bis 7 zur Behandlung der Schizophrenie.

## Claims

1. Compound of formula (I) : 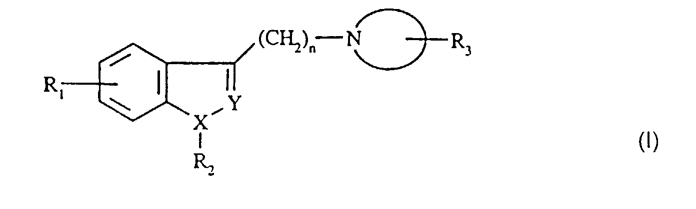 wherein :
R₁ represents a hydrogen atom, a halogen atom, or a linear or branched (C₁-C₆)alkyl, linear or branched (C₁-C₆)alkoxy, trihalomethyl or hydroxy group,
R₂ represents a hydrogen atom, a linear or branched (C₁-C₆)alkyl group or a phenyl group (unsubstituted or substituted by one or more halogen atoms or alkyl, alkoxy, hydroxy or trihalomethyl group), 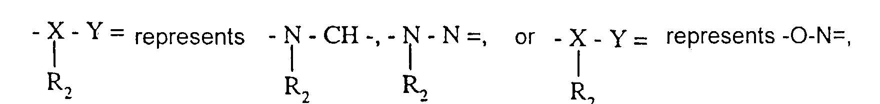 1 ≤ n ≤ 6, 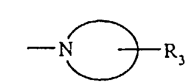 represents any one of the following groups : 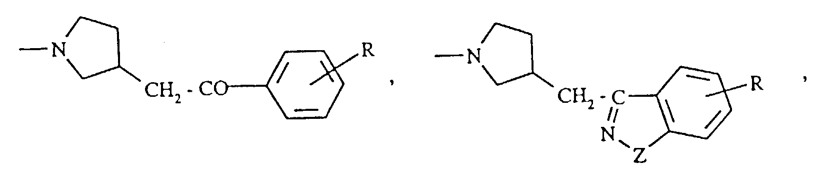 or 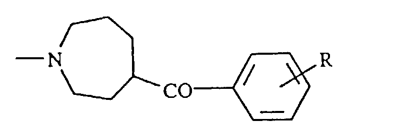 wherein :
R represents a hydrogen atom, a halogen atom or a linear or branched (C₁-C₆)alkyl, linear or branched (C₁-C₆)alkoxy, hydroxy or trihalomethyl group,
Z represents an oxygen atom, a sulphur atom or an -NH- group,
their isomers, and addition salts thereof with a pharmaceutically acceptable acid or base.

2. Compound of formula (I) according to claim 1, characterised in that 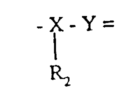 represents -NH-CH=.

3. Compound of formula (I) according to claim 1, characterised in that n is 2.

4. Compound of formula (I) according to claim 1, characterised in that n is 3.

5. Compound of formula (I) according to claim 1, characterised in that 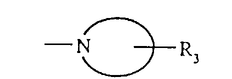 represents

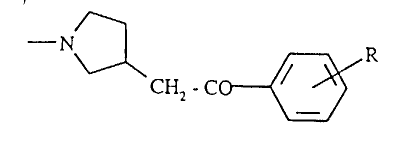

6. Compound of formula (I) according to claim 1, characterised in that 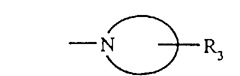 represents

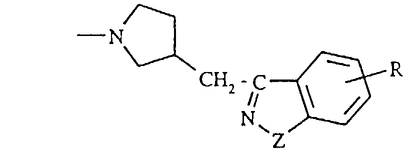

7. Compound of formula (I) according to claim 1, characterised in that 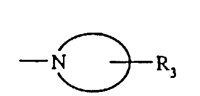 represents

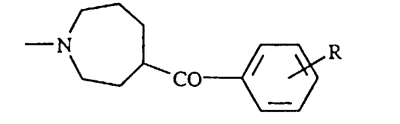

8. Process for the preparation of compounds of formula (I) according to claim 1, characterised in that there is used as starting material an alcohol of formula (II) : 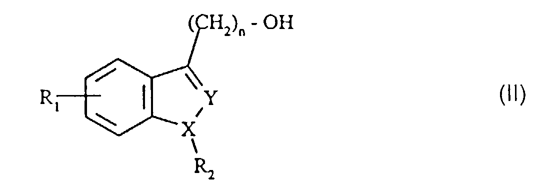 wherein R₁, R₂, X, Y and n are as defined for formula (I),
which is converted into the corresponding bromine compound using carbon tetrabromide in the presence of triphenylphosphine,
to yield a compound of formula (III) : 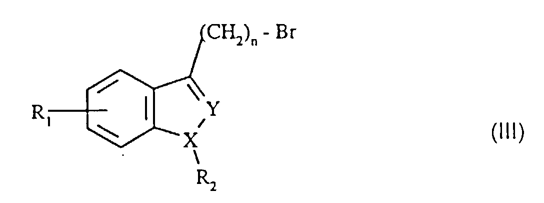 wherein R₁, R₂, X, Y and n are as defined for formula (I),
which is reacted with an amine of formula (IV) : 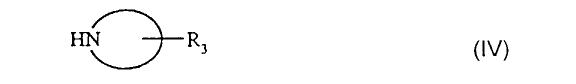 wherein R₃ is as defined for formula (I),
to yield a compound of formula (I),
- which, if necessary, may be purified according to a conventional purification technique,
- which, if appropriate, is separated into its isomers according to a conventional separation technique,
- which, if desired, is converted into its addition salts with a pharmaceutically acceptable base.

9. Pharmaceutical composition comprising as active ingredient at least one compound according to any one of claims 1 to 7, alone or in combination with one or more pharmaceutically acceptable, inert, non-toxic carriers.

10. Pharmaceutical composition according to claim 9 containing at least one active ingredient according to any one of claims 1 to 7 for use in the treatment of schizophrenia.
